# EUROPÄISCHE PATENTSCHRIFT

(11) **EP 1 513 824 B1**
(45) Veröffentlichungstag und Bekanntmachung des Hinweises auf die Patenterteilung: **01.10.2008**
(21) Anmeldenummer: 03755103.3
(22) Anmeldetag: 15.05.2003
(51) Int. Cl.: C07D 307/68, A01N 43/08

(54) **FURANCARBOXAMIDE**
FURANCARBOXAMIDES
FURANE CARBOXAMIDES

(30) Priorität: 23.05.2002 DE 10222884
(43) Veröffentlichungstag der Anmeldung: 16.03.2005
(73) Patentinhaber: Bayer CropScience AG, 40789 Monheim (DE)
(72) Erfinder: RIECK, Heiko, F-69009 Lyon (FR); DUNKEL, Ralf, 69001 Lyon (FR); ELBE, Hans-Ludwig, 42329 Wuppertal (DE); WACHENDORFF-NEUMANN, Ulrike, 56566 Neuwied (DE); MAULER-MACHNIK, Astrid, 42799 Leichlingen (DE); KUCK, Karl-Heinz, 40764 Langenfeld (DE)
(86) Internationale Anmeldenummer: PCT/EP2003/005105
(87) Internationale Veröffentlichungsnummer: WO 2003/099803

(56) Entgegenhaltungen:
- WO-A-02/08197
- GB-A- 1 303 844
- JP-A- 2001 302 605

## Beschreibung

Die vorliegende Erfindung betrifft neue Furancarboxamide, mehrere Verfahren zu deren Herstellung und deren Verwendung zur Bekämpfung von unerwünschten Mikroorganismen.

Es ist bereits bekannt, dass zahlreiche Furäncarboxamide fungizide Eigenschaften besitzen (vgl. z.B. EP-A 0 545 099, EP-A 0 591 699, EP-A 0 597 336, EP-A 0 755 927, DE-OS 24 09 011, DE-OS 20 06 472, DE-OS 20 06 471, JP-A 2001-302605, JP-A 9-255675 und JP-A 8-176112). So sind z.B. bereits die Furancarboxamide *N*-(1,1'-Biphenyl-2-yl)-2,4,5-trimethyl-3-furamid (EP-A 0 545 099), *N*-(1,1'-Biphenyl-2-yl)-2-methyl-3-furamid (DE-OS 20 06 472), *N*-(6-Chlor-4'-fluor-1,1'-biphenyl-2-yl)-2-(trifluormethyl)-3-furamid (JP-A 2001-302605) und *N*-Acetyl-*N*-(1,1'-biphenyl-2-yl)-2-methyl-3-furamid (JP-A 8-176112) bekannt. Die Wirksamkeit dieser Stoffe ist gut, lässt aber in manchen Fällen, z.B. bei niedrigen Aufwandmengen zu wünschen übrig.

Es wurden nun neue Furancarboxamide der Formel (I) 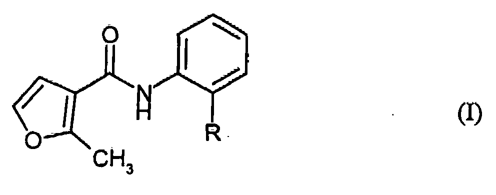 gefunden, in welcher
- R: für eine der folgenden Gruppierungen steht, 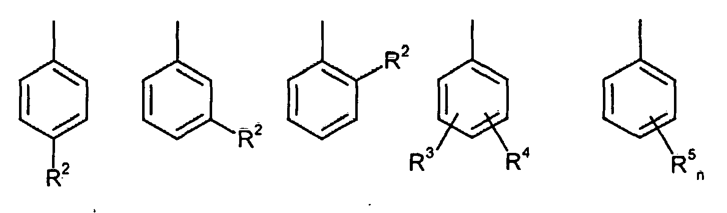
- R²: für Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogenalkylthio mit jeweils 1 bis 13 Fluor-, Chlor- und/oder Bromatomen steht,
- R³ und R⁴: unabhängig voneinander für Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogenalkylthio mit jeweils 1 bis 13 Fluor-, Chlor- und/oder Bromatomen stehen,
- n: für 3, 4 oder 5 steht und
- R⁵: für gleiche oder verschiedene Reste aus der Reihe Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogenalkylthio mit jeweils 1 bis 13 Fluor-, Chlor- und/oder Bromatomen steht.

Weiterhin wurde gefunden, dass man Furancarboxamide der Formel (I) erhält, indem man
a) Carbonsäure-Derivate der Formel (II) 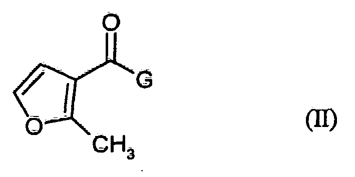 in welcher
   - G: für Halogen, Hydroxy öder C₁-C₆-Alkoxy steht, mit Anilin-Derivaten der Formel (III) 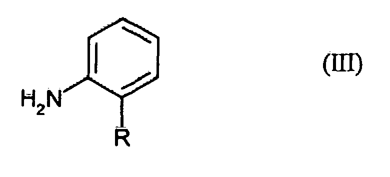
   in welcher
   R und m die oben angegebenen Bedeutungen haben,
   gegebenenfalls in Gegenwart eines Katalysators, gegebenenfalls in Gegenwart eines Säurebindemittels und gegebenenfalls in Gegenwart eines Verdünnungsmittels umsetzt,
   oder
b) Carboxamid-Derivate der Formel (IV) 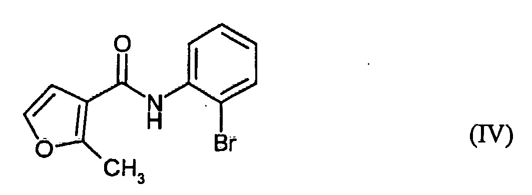 mit Boronsäure-Derivaten der Formel (V) 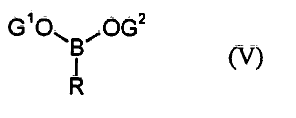 in welcher
   - R: die oben angegebenen Bedeutungen hat und
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt,
   oder
c) Carboxamid-Boronsäure-Derivate der Formel (VI) 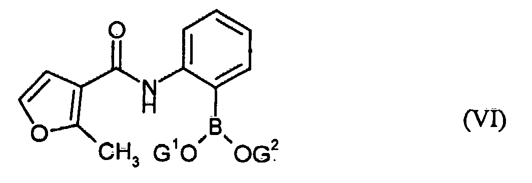 in welcher
   - G¹ und G²: jeweils für Wasserstoff oder zusammen für Tetramethylethylen stehen,
   mit Phenyl-Derivaten der Formel (VII) 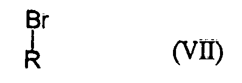 in welcher
   R die oben angegebenen Bedeutungen hat,
   in Gegenwart eines Katalysators, gegebenenfalls in Gegenwart eines Säurebindemittels und gegebenenfalls in Gegenwart eines Verdünnungsmittels umsetzt,
   oder
d) Carboxamid-Derivate der Formel (IV) 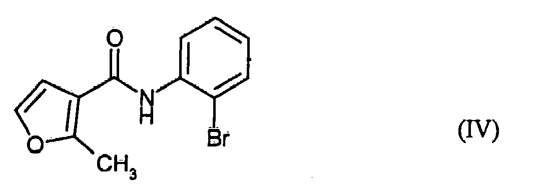 mit Phenyl-Derivaten der Formel (VII) 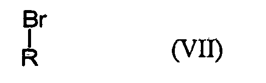
in welcher
R die oben angegebenen Bedeutungen hat,
in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

Schließlich wurde gefunden, dass die neuen Furancarboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Furancarboxamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Furancarboxamide sind durch die Formel (I) allgemein definiert.

Bevorzugt sind Furancarboxamide der Formel (I), in welcher
- R: für eine der folgenden Gruppierungen steht, 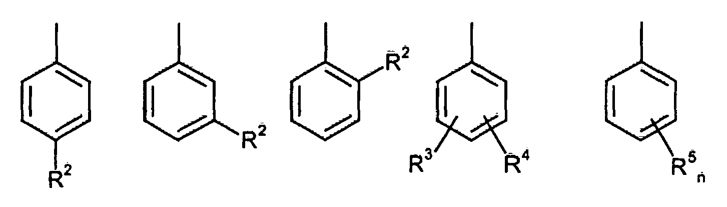
- R²: für Fluor, Chlor, Brom, Iod, C₁-C₆-Alkyl, C₁-C₆-Alkoxy, C₁-C₆-Alkylthio, C₁-C₄-Halogenalkyl, C₁-C₄-Halogenalkoxy, C₁-C₄-Halogenalkylthio mit jeweils 1 bis 9 Fluor-, Chlor und/oder Bromatomen steht,
- R²: außerdem für Cyano steht,
- R³ und R⁴: unabhängig voneinander für Fluor, Chlor, Brom, Iod, G₁-C₆-Alkyl, C₁-C₆-Alkoxy, C₁-C₆-Alkylthio, C₁-C₄-Halogenalkyl, C₁-C₄-Halogenalkoxy, C₁-C₄-Halogenalkylthio mit jeweils 1 bis 9 Fluor-, Chlor und/oder Bromatomen stehen,
- n: für 3, 4 oder 5 steht und
- R⁵: für gleiche oder verschiedene Reste aus der Reihe Fluor, Chlor, Brom, Iod, C₁-C₆-Alkyl, C₁-C₆-Alkoxy, C₁-C₆-Alkylthio, C₁-C₄-Halogenalkyl, C₁-C₄-Halogenalkoxy, C₁-C₄-Halogenalkylthio mit jeweils 1 bis 9 Fluor-, Chlor und/oder Bromatomen steht.

Besonders bevorzugt sind Furancarboxamide der Formel (I), in welcher
- R: für eine der folgenden Gruppierungen steht, 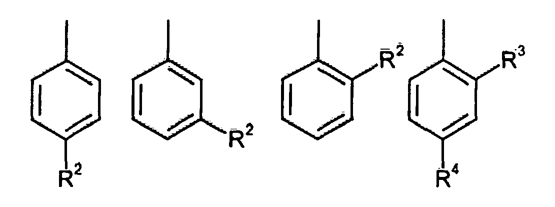 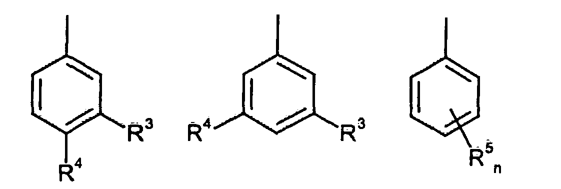
- R²: für Fluor, Chlor, Brom, Methyl, Ethyl, n-Propyl, i-Propyl, n-Butyl, i-Butyl, s-Butyl, t-Butyl, Methoxy, Ethoxy, Methylthiö, Ethylthio, Trichlormethyl, Tri-fluormethyl, Difluormethyl, Difluorchlormethyl, Difluormethoxy, Trifluormethoxy, Trifluormethylthio, Difluorchlormethylthio steht,
- R²: außerdem für Cyano steht,
- R³ und R⁴: unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluorethyl, Difluormethyl, Difluorchlormethyl, Difluormethoxy, Trifluormethoxy, Trifluormethylthio, Difluorchlormethylthio stehen,
- n: für 3 oder 4 steht und
- R⁵: für gleiche oder verschiedene Reste aus der Reihe Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio steht.

Ganz besonders bevorzugt sind Furancarboxamide der Formel (I), in welcher
- R: für eine der folgenden Gruppierungen steht, 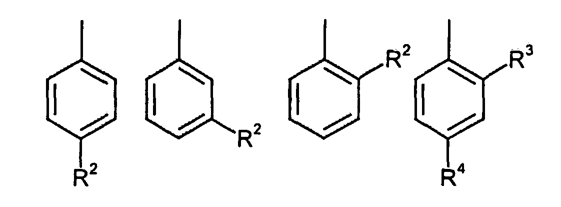 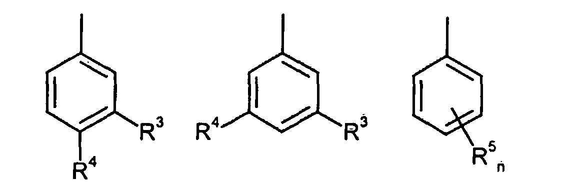
- R²: für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio steht,
- R²: außerdem für Cyano steht,
- R³ und R⁴: unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio stehen,
- n: für 3 steht und
- R⁵: für gleiche oder verschiedene Reste aus der Reihe Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio steht.

Insbesondere bevorzugt sind Furancarboxamide der Formel (I), in welcher
- R: für eine der folgenden Gruppierungen steht, 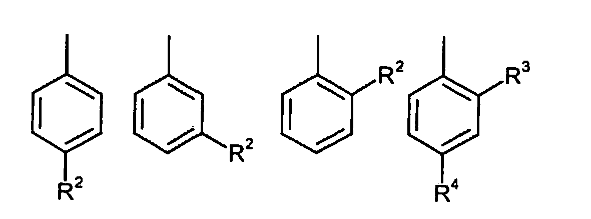 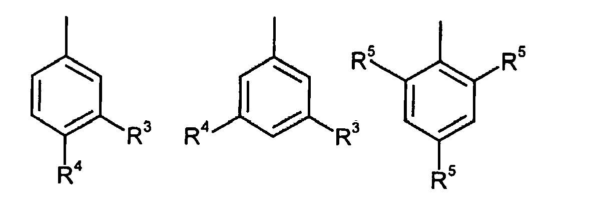
- R²: für Fluor, Chlor, Brom, t-Butyl, Methoxy, Methylthio, Trichlormethyl, Trifluormethyl, Trifluormethoxy, Trifluormethylthio steht,
- R²: außerdem für Cyano steht,
- R³ und R⁴: unabhängig voneinander für Fluor, Chlor, Methyl, Methoxy, Methylthio, Trifluormethyl, Trifluormethoxy, Trifluormethylthio stehen,
- R⁵: für gleiche oder verschiedene Reste aus der Reihe Fluor, Chlor, Methyl, Methoxy, Methylthio, Trifluormethyl, Trifluormethoxy, Trifluormethylthio steht.

Außerdem hervorgehoben sind Verbindungen der Formel (I), in welcher R für eine der folgenden Gruppierungen 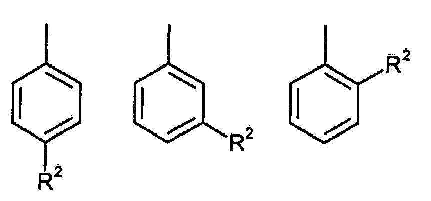 steht und R² die oben angegebenen allgemeinen, bevorzugten, besonders bevorzugten, ganz besonders bevorzugten und/oder insbesondere ganz besonders bevorzugten Bedeutungen hat.

Außerdem hervorgehoben sind Verbindungen der Formel (I), in welcher R für eine der folgenden Gruppierungen 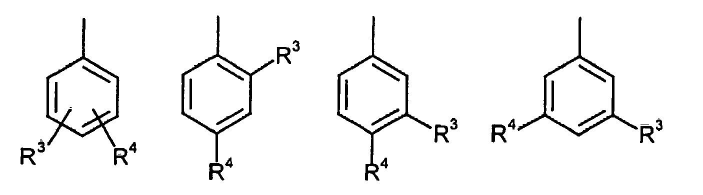 steht und R³ und R⁴ die oben angegebenen allgemeinen, bevorzugten, besonders bevorzugten, ganz besonders bevorzugten und/oder insbesondere ganz besonders bevorzugten Bedeutungen haben.

Außerdem hervorgehoben sind Verbindungen der Formel (I), in welcher R³ und R⁴ unabhängig voneinander für Fluor, Chlor oder Brom stehen.

Außerdem hervorgehoben sind Verbindungen der Formel (I-a) 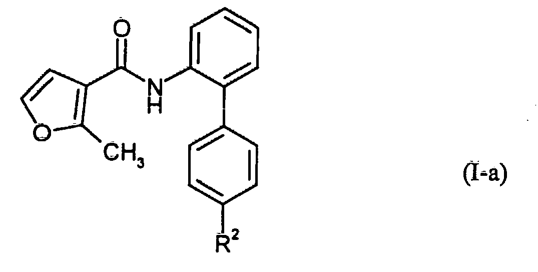 in welcher
- R²: für Fluor, Chlor, Brom, Methyl, Ethyl, n-Pröpyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio steht,
- R²: außerdem für Cyano steht.

Außerdem hervorgehoben sind Verbindungen der Formel (I-b) 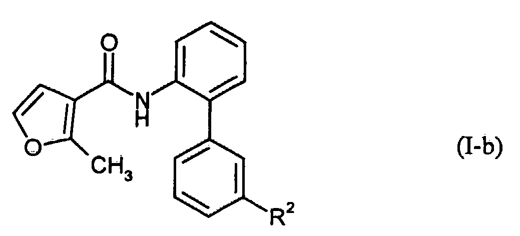 in welcher
- R²: für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio steht.

Außerdem hervorgehoben sind Verbindungen der Formel (I-c) 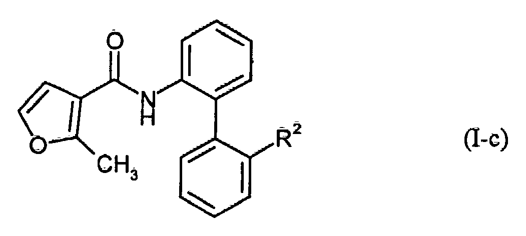 in welcher
- R²: für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthiö, Trichlormethyl, Tri-fluormethyl, Difluormethyl, Difluorchlormethyl, Difluormethoxy, Trifluormethoxy, Trifluormethylthio, Difluorchlormethylthio steht.

Außerdem hervorgehoben sind Verbindungen der Formel (I-d) 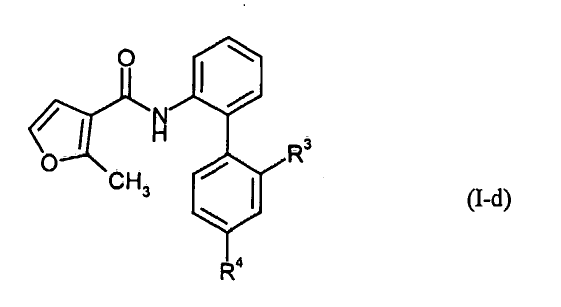 in welcher
- R³ und R⁴: unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen.

Außerdem hervorgehoben sind Verbindungen der Formel (I-e) 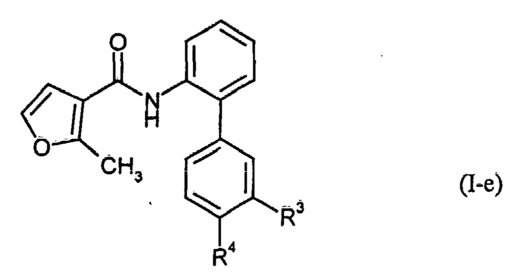 in welcher
- R³ und R⁴: unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen.

Außerdem hervorgehoben sind Verbindungen der Formel (I-f) 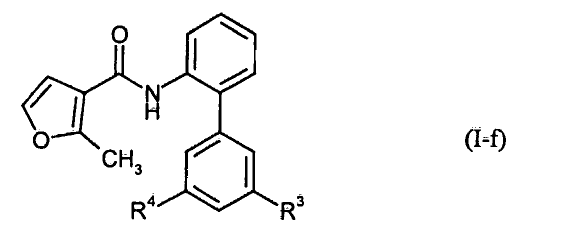 in welcher
- R³ und R⁴: unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Verwendet man 2-Methyl-furan-3-carbonylchlorid und 4'-Chlor-2'-fluor-1,1'-biphenyl-2-amin als Ausgangsstoffe, so kann der Verlauf des erfindungsgemäßen Verfahrens (a) durch das folgende Formelschema veranschaulicht werden.

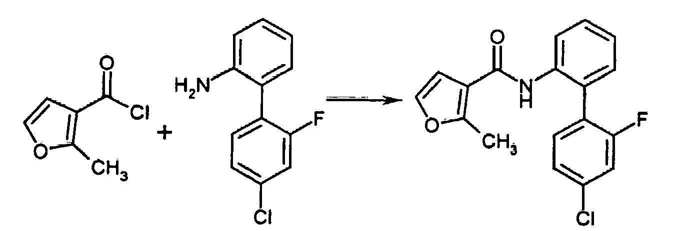

Verwendet man *N*-(2-Bromphenyl)-2-methyl-3-furamid und 4-Chlor-2-fluorphenylboronsäure als Ausgangsstoffe sowie einen Katalysator, so kann der Verlauf des erfindungsgemäßen Verfahrens (b) durch das folgende Formelschema veranschaulicht werden.

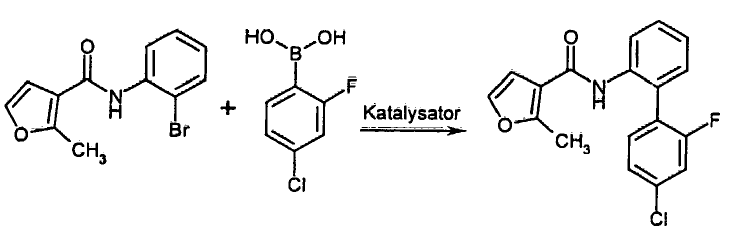

Verwendet man 2-{[(2-Methyl-furan-3-yl)carbonyl]amino}phenylboronsäure und 1-Brom-4-chlor-2-fluorbenzol als Ausgangsstoffe sowie einen Katalysator, so kann der Verlauf des erfindungsgemäßen Verfahrens (c) durch das folgende Formelschema veranschaulicht werden.

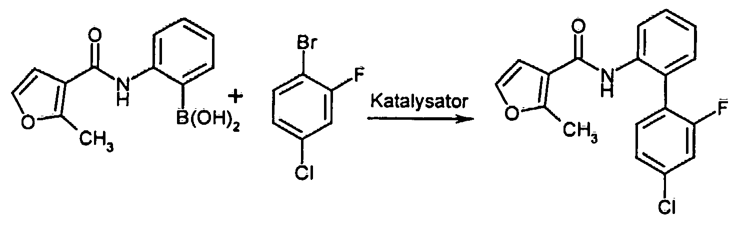

Verwendet man *N*-(2-Bromphenyl)-2-methyl-3-furamid und 1-Brom-4-chlor-2-fluorbenzol als Ausgangsstoffe sowie einen Katalysator und 4,4,4',4',5,5,5',5'-Octamethyl-2,2'-bis-1,3,2-dioxaborolan, so kann der Verlauf des erfindungsgemäßen Verfahrens (d) durch das folgende Formelschema veranschaulicht werden.

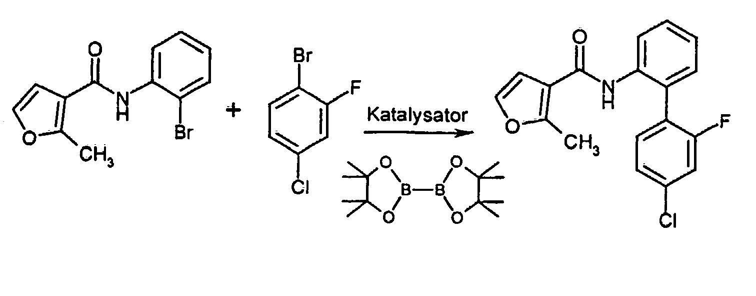

### Erläuterung der Verfahren und Zwischenprodukte

Die bei der Durchführung des erfindungsgemäßen Verfahrens (a) als Ausgangsstoffe benötigten Carbonsäure-Derivate sind durch die Formel (II) allgemein definiert. In dieser Formel steht G bevorzugt für Chlor, Brom, Hydroxy, Methoxy oder Ethoxy, besonders bevorzugt für Chlor, Hydroxy oder Methoxy, ganz besonders bevorzugt für Chlor.

Die Carbonsäure-Derivate der Formel (II) sind bekannt oder lassen sich nach bekannten Verfahren herstellen (vgl. EP-A 0 545 099 und EP-A 0 589 313).

Die bei der Durchführung des erfindungsgemäßen Verfahrens (a) als Reaktionskomponenten benötigten Anilin-Derivate sind durch die Formel (III) allgemein definiert. In dieser Formel hat R vorzugsweise diejenige Bedeutung, die bereits im Zusammenhang mit der Beschreibung der erfindungsgemäßen Stoffe der Formel (I) als bevorzugt, besonders bevorzugt, ganz besonders bevorzugt bzw. insbesondere bevorzugt für diese Reste bzw. diese Indices genannt wurde.

Die Anilin-Derivate der Formel (III) sind bekannt und/oder lassen sich teilweise nach bekannten Methoden herstellen (vgl. EP-A 0 545 099 und EP-A 0 589 301). Man erhält Anilin-Derivate der Formel (III) beispielsweise, indem man

### e) 2-Halogenanilin-Derivate der allgemeinen Formel (VIII)

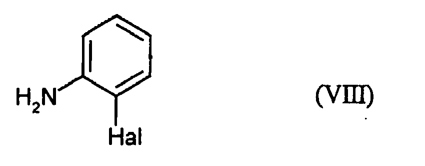

in welcher
- Hal: für Halogen steht, mit Boronsäure-Derivaten der Formel (V) 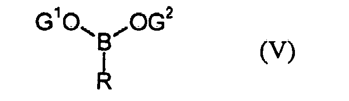
in welcher
R die oben angegebenen Bedeutungen hat,
gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt
oder

### f) Anilinboronsäuren der Formel (IX)

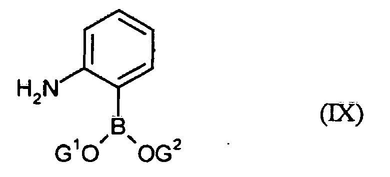

in welcher
G¹ und G² die oben angegebenen Bedeutungen haben
mit Phenyl-Derivaten der Formel (VII) 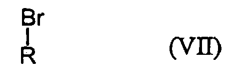 in welcher
R die oben angegebenen Bedeutungen hat,
gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung des erfindungsgemäßen Verfahrens (e) als Reaktionskomponenten benötigten 2-Halogenanilin-Derivate sind durch die Formel (VIII) allgemein definiert. Hal steht vorzugsweise für Fluor, Chlor oder Brom, besonders bevorzugt für Chlor oder Brom.

Die 2-Halogenanilin-Derivate der Formel (VIII) sind bekannt und/oder lassen sich aus den entsprechenden Nitroverbindungen durch Reduktion herstellen.

Die zur Durchführung des erfindungsgemäßen Verfahrens (e) außerdem als Ausgangsstoffe benötigten Boronsäure-Derivate der Formel (V) werden unten im Zusammenhang mit dem erfindungsgemäßen Verfahren (b) näher erläutert.

Die bei der Durchführung des erfindungsgemäßen Verfahrens (f) als Reaktionskomponenten benötigten Anilinboronsäuren sind durch die Formel (IX) allgemein definiert. G¹ und G² stehen bevorzugt jeweils für Wasserstoff oder zusammen für Tetramethylethylen.

Die Anilinboronsäuren der Formel (IX) sind bekannt und/öder lassen sich nach bekannten Methoden erhalten.

Die zur Durchführung des erfindungsgemäßen Verfahrens (f) außerdem als Ausgangsstoffe benötigten Phenyl-Derivate der Formel (VII) werden unten im Zusammenhang mit dem erfindungsgemäßen Verfahren (d) näher erläutert.

Die bei der Durchführung der erfindungsgemäßen Verfahren (b) und (d) als Ausgangsstoffe benötigten Carboxamid-Derivate sind durch die Formel (IV) allgemein definiert.

Die Carboxamid-Derivate der Formel (IV) sind bekannt oder lassen sich nach bekannten Verfahren herstellen (z.B. aus einem Carbonsäure-Derivat der Formel (III) und einem 2-Bromanilin-Derivat).

Die bei der Durchführung der erfindungsgemäßen Verfahren (b) und (e) als Ausgangsstoffe benötigten Boronsäure-Derivate sind durch die Formel (V) allgemein definiert. In dieser Formel hat R vorzugsweise diejenigen Bedeutungen, die bereits im Zusammenhang mit der Beschreibung der erfindungsgemäßen Stoffe der Formel (I) als bevorzugt, besonders bevorzugt, ganz besonders bevorzugt bzw. insbesondere bevorzugt für diese Reste bzw. diese Indices genannt wurden. G¹ und G² stehen bevorzugt jeweils für Wasserstoff oder zusammen für Tetramethylethylen.

Die Boronsäure-Derivate der Formel (V) sind bekannt und/oder lassen sich nach bekannten Verfahren herstellen (vgl. z.B. WO 01/90084 und US 5,633,218). Sie werden beispielsweise erhalten, indem man

### g) Phenyl-Derivate der Formel (VII)

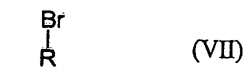

in welcher
R die oben angegebenen Bedeutungen hat,
mit Borsäureestern der Formel (X)

B(OR⁶)₃ (X)

in welcher
R⁶ für C₁-C₄-Alkyl steht,
in Gegenwart von Magnesium gegebenenfalls in Gegenwart eines Verdünnungsmittels (z.B. Tetrahydrofuran) umsetzt.

Die bei der Durchführung des erfindungsgemäßen Verfahrens (g) als Reaktionskomponenten benötigten Borsäureester sind durch die Formel (X) allgemein definiert. In dieser Formel steht R⁶ bevorzugt für Methyl, Ethyl, n- oder i-Propyl, besonders bevorzugt für Methyl oder Ethyl.

Die Borsäureester der Formel (X) sind bekannte Synthesechemikalien.

Die bei der Durchführung der erfindungsgemäßen Verfahrens (c) als Reaktionskomponenten benötigten Carboxamid-Boronsäure-Derivate sind durch die Formel (VI) allgemein definiert. G¹ und G² stehen bevorzugt jeweils für Wasserstoff oder zusammen für Tetramethylethylen.

Die Carboxamid-Boronsäure-Derivate der Formel (VI) sind bekannt und/oder lassen sich nach bekannten Verfahren herstellen.

Die bei der Durchführung der erfindungsgemäßen Verfahren (c), (d), (f) und (g) als Ausgangsstoffe benötigten Phenyl-Derivate sind durch die Formel (VII) allgemein definiert. In dieser Formel steht R vorzugsweise für diejenigen Bedeutungen, die bereits in Zusammenhang mit der Beschreibung der erfindungsgemäßen Stoffe der Formel (I) für diese Reste als bevorzugt, besonders bevorzugt, ganz besonders bevorzugt bzw. insbesondere bevorzugt genannt wurden.

Die Phenyl-Derivate der Formel (VII) sind bekannt oder lassen sich nach bekannten Verfahren herstellen (vgl. Synth. Commun. 2000, 30, 665-669, Synth. Commun. 1999, 29, 1697-1701).

Als Säurebindemittel kommen bei der Durchführung der erfindungsgemäßen Verfahren (a), (b), (c), (d), (e) und (f) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert.

Als Verdünnungsmittel kommen bei der Durchführung der erfindungsgemäßen Verfahren (a), (b), (c), (d), (e) und (f) jeweils alle üblichen inerten, organischen Solventien in Frage. Vorzugsweise verwendbar sind gegebenenfalls Halogenierte aliphatische, alicyclische oder aromatische Kohlenwasserstoffe, wie Petrolether, Hexan, Heptan, Cyclohexen, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Die Reaktionstemperaturen können bei der Durchführung der erfindungsgemäßen Verfahren (a), (b), (c), (d), (e) und (f) jeweils in einem größeren Bereich variiert werden. Im allgemeinen arbeitet man bei Temperaturen zwischen 0°C und 140°C, vorzugsweise zwischen 10°C und 120°C.

Bei der Durchführung der erfindungsgemäßen Verfahren (a), (b), (c), (d), (e) und (f) arbeitet man im allgemeinen jeweils unter Atmosphärendruck. Es ist aber auch möglich, jeweils unter erhöhtem oder vermindertem Druck zu arbeiten.

Bei der Durchführung des erfindungsgemäßen Verfahrens (a) setzt man auf 1 Mol an Carbonsäure-Derivat der Formel (II) im allgemeinen 1 Mol oder auch einen Überschuss an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (b) setzt man auf 1 Mol an Carboxamid-Derivat der Formel (IV) im allgemeinen 1 Mol oder auch einen Überschuss an Boronsäure-Derivat der Formel (V) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (c) setzt man auf 1 Mol an Carboxamid-Boronsäure-Derivat der Formel (VI) im allgemeinen 1 Mol oder auch einen Überschuss an Phenyl-Derivat der Formel (VII) sowie 1 bis 10 Mol an Säurebindemittel und 0.5 bis 5 Molprozent eines Katalysators ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (d) setzt man auf 1 Mol an Carboxamid-Derivat der Formel (IV) im allgemeinen 1 Mol oder auch einen Überschuss an Phenyl-Derivat der Formel (VII) sowie 1 bis 5 Mol an Säurebindemittel ein, sowie 1 bis 5 Mol eines Katalysators. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora=Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrags. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden.

Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Dichlofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, ethirimol, Etridiazol,
Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin, Hexachlorobenzol, Hexaconazol, Hymexazol,
Imazalil, Imibenconazol, Iminoctadin, Immoctadinealbesilat, Iminoctadinetriacetat, Iodocarb, Ipconazol, Iprobenfos (IBP), Iprodione, Iprovalicarb, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Pölyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB), Quinoxyfen
Schwefel und Schwefel-Zubereitungen, Spiroxamine
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol, Validamycin A, Vinclozolin, Viniconazol,
Zarilamid, Zineb, Ziram sowie
Dagger G, OK-8705, OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol;
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat, cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,
4-[(3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin, Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben, Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine, Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn, Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fösmethilan, Fosthiazate, Fubfenprox, Furathiocarb, Granuloseviren Halofenozide, HCH, Heptenophos, Hexaflumuron, Hexythiazox, Hydroprene, Imidacloprid, Indoxacarb, Isazofos, Isofenphos, Isoxathion, Ivermectin, Kempolyederviren, Lambda-cyhalothrin, Lufenuron Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos, Naled, Nitenpyram, Nithiazine, Novaluron Omethoat, Oxamyl, Oxydemethon M Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen, Quinalphos, Ribavirin, Salithion, Sebufos, Silafluofen, Spinosad, Spirodiclofen, Sulfotep, Sulprofos, Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb, Vamidothion, Vaniliprole, Verticillium lecanii YI 5302, Zeta-cypermethrin, Zolaprofos (1R-cis)-[5-(Phenylmethyl)-3-furanyl]-methyl-3-[(dihydro-2-oxo-3(2H)-furanyliden)-methyl]-2,2-dimethylcyclopropancarboxylat (3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin 2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion 2-Chlor-N-[[[4-(1-phenylethoxy)-phenyl]-amino]-carbonyl]-benzamid 2-Chlor-N-[[[4-(2,2-dichlor-1,1-difluorethoxy)-phenyl]-amino]-carbonyl]-benzamid 3-Methylphenyl-propylcarbamat 4-[4-(4-Ethoxyphenyl)-4-methylpentyl]-1-fluor-2-phenoxy-benzol 4-Chlor-2-(1,1-dimethylethyl)-5-[[2-(2,6-dimethyl-4-phenoxyphenoxy)ethyl]thio]-3(2H)-pyridazinon 4-Chlor-2-(2-chlor-2-methylpropyl)-5-[(6-iod-3-pyridinyl)methoxy]-3(2H)-pyridazinon 4-Chlor-5-[(6-chlor-3-pyridinyl)methoxy]-2-(3,4-dichlorphenyl)-3(2H)-pyridazinon Bacillus thuringiensis strain EG-2348 Benzoesäure [2-benzoyl-1-(1,1-dimethylethyl)-hydrazid Butansäure 2,2-dimethyl3-(2,4-dichlorphenyl)-2-oxo-1-oxaspiro[4.5]dec-3-en-4-yl-ester [3-[(6-Chlor-3-pyridinyl)methyl]-2-thiazolidinyliden]-cyanamid Dihydro-2-(nitromethylen)-2H-1,3-thiazine-3(4H)-carboxaldehyd Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin N-(4-Chlorphenyl)-3-[4-(difluormethoxy)phenyl]-4,5-dihydro-4-phenyl-1H-pyrazol-1-carboxamid N-[(2-Chlor-5-thiazolyl)methyl]-N'-methyl-N"-nitro-guanidin N-Methyl-N'-(1-methyl-2-propenyl)-1,2-hydrazindicarbothioamid N-Methyl-N'-2-propenyl-1,2-hydrazindicarbothioamid O,O-Diethyl-[2-(dipropylamino)-2-oxoethyl]-ethylphosphoramidothioat N-Cyanomethyl-4-trifluormethyl-nicotinamid 3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze ( z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstizmsbedingungen (Böden, Klima, Vegetationsperiode, Ernährung) können durch die erfindungsgemäße Behandlung auch überadditive ("synergistische") Effekte auftreten. So sind beispielsweise erniedrigt Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten, gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucoton^{®} (Baumwolle) und NewLeaf^{®} (Kartoffel) vertrieben werden. Als Beispiele für Herbizid tolerante Pflanzen seien Maissorten, Baumwöllsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

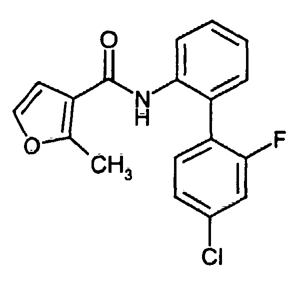

Zu einer Suspension von 207 mg Kaliumcarbonat in 25 ml Acetonitril werden 332 mg 4'-Chlor-2'-fluor-1,1'-biphenyl-2-amin und 216 mg 2-Methyl-3-furoylchlorid getropft. Das Reaktionsgemisch wird 10 h gerührt. Zur Aufarbeitung wird die Reaktionslösung mit 20 ml gesättigter Ammoniumchlorid-Lösung versetzt und das Gemisch mit Ethylacetat extrahiert. Die organischen Phasen werden mit Natriumsulfat getrocknet und eingeengt. Der Rückstand wird an Kieselgel (Cyclohexan/Ethylacetat 2:1) chromatographiert.

Man erhält 290 mg (57 %) an *N*-(4'-Chlor-2'-fluor-1,1'-biphenyl-2-yl)-2-methyl-3-furamid mit dem logP (pH 2,3) = 3,38.

Analog dem zuvor beschriebenen Beispiel 1 sowie entsprechend den allgemeinen Verfahrensbeschreibungen werden auch die in der folgenden Tabelle 1 aufgeführten Furancarboxamide der Formel (I) hergestellt.

**Tabelle 1**

| | | |
|---|---|---|
| 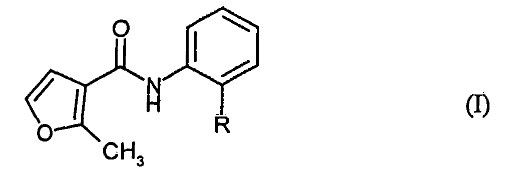 | | |

| **Bsp.** | **R** | **logP (pH2,3)/Fp.** |
|---|---|---|
| 2 | 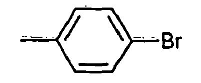 | 3,71 |
| 3 | 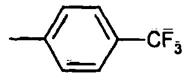 | 3,53 128°C |
| 4 | 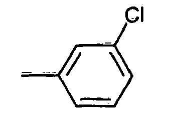 | 3,58 |
| 5 | 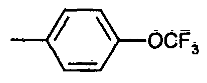 | 3,91 |
| 6 | 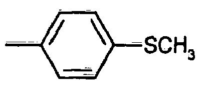 | 3,63 |
| 7 | 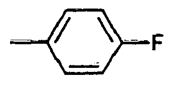 | 3,27 |
| 8 | 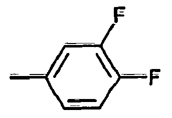 | 3,22 |
| 9 | 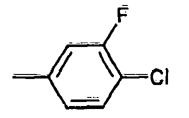 | 3,43 |
| 10 | 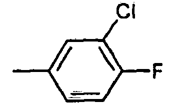 | 3,46 |
| 11 | 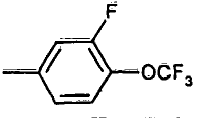 | 3,81 |
| 12 | 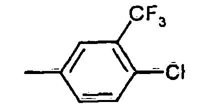 | 3,81 |
| 13 | 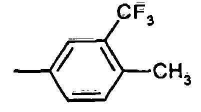 | 3,90 |
| 14 | 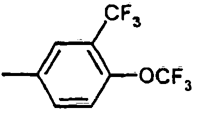 | 4,08 |
| 15 | 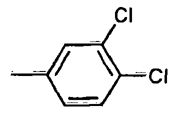 | 3,73 |
| 16 | 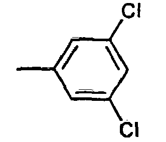 | 3,90 |
| 17 | 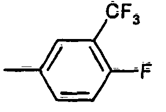 | 3,55 |
| 18 | 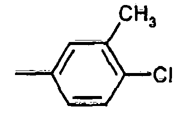 | 3,94 |
| 19 | 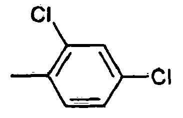 | 3,75 |
| 20 | 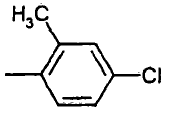 | 4,08 |
| 21 | 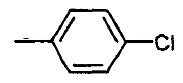 | 3,45 |
| 22 | 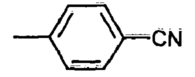 | 2,56 |
| 23 | 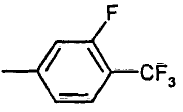 | 3,54 115°C |
| 24 | 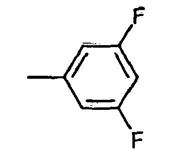 | 3,16 143°C |
| 25 | 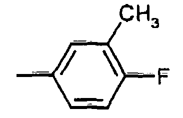 | 3,55 |
| 26 | 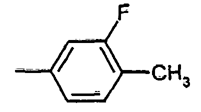 | 3,52 |
| 27 | 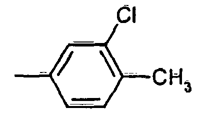 | 3,84 |
| 28 | 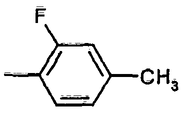 | 3,36 |
| 29 | 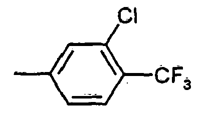 | 3,78 113-115°C |

Die Bestimmung der in den Herstellungsbeispielen angegebenen IogP-Werte erfolgte gemäß EEC-Directive 70/831 Annex V.A8 durch HPLC (High Performance Liquid Chromatography) an einer Phasenumkehrsäule (C 18). Temperatur: 43°C.
Eluenten für die Bestimmung im sauren Bereich: 0,1 % wässrige Phosphorsäure, Acetonitril; linearer Gradient von 10 % Acetonitril bis 90 % Acetonitril. Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).
Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| **Podosphaera-Test (Apfel) / protektiv** | | | |
|---|---|---|---|
| Wirkstoff | | Aufwandmenge an Wirkstoff in g/ha | Wirkungsgrad in % |
| 3 | 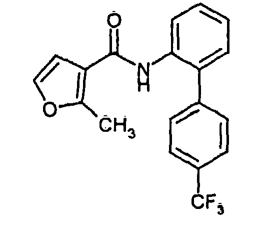 | 100 | 93 |
| 4 | 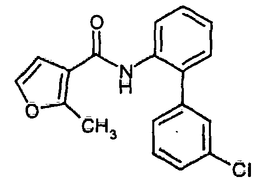 | 100 | 100 |
| 5 | 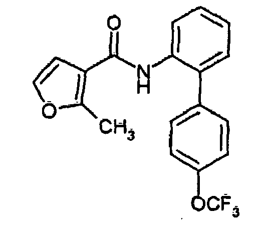 | 100 | 93 |
| 6 | 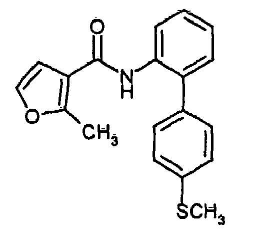 | 100 | 92 |
| 8 | 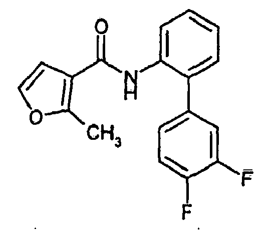 | 100 | 85 |
| 9 | 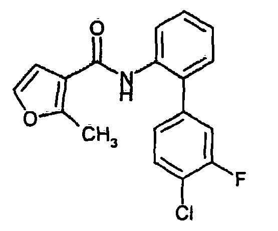 | 100 | 97 |
| 10 | 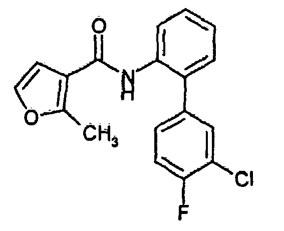 | 100 | 100 |
| 15 | 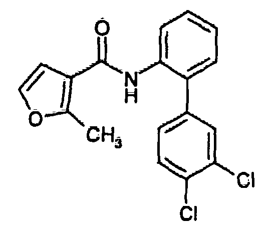 | 100 | 100 |

### Beispiel B

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B**

| **Venturia - Test (Apfel) / protektiv** | | | |
|---|---|---|---|
| Wirkstoff | | Aufwandmenge an Wirkstoff in g/ha | Wirkungsgrad in % |
| 2 | 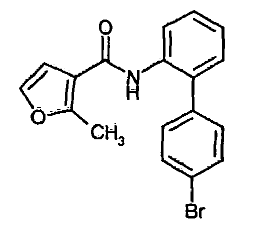 | 100 | 100 |
| 3 | 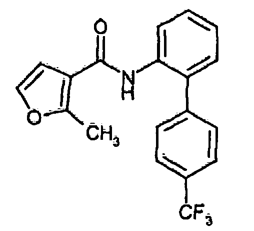 | 100 | 100 |
| 4 | 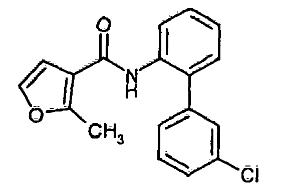 | 100 | 100 |
| 5 | 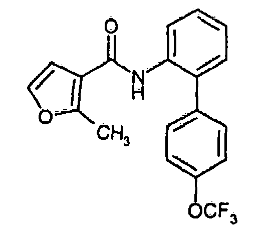 | 100 | 100 |
| 6 | 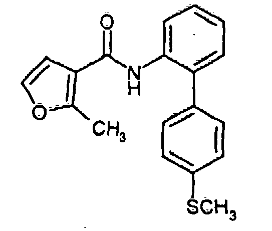 | 100 | 100 |
| 8 | 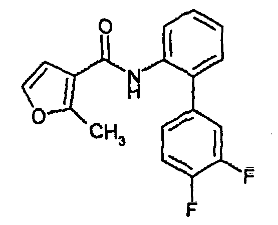 | 100 | 100 |
| 9 | 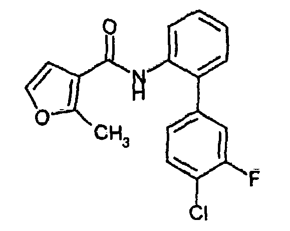 | 100 | 100 |
| 10 | 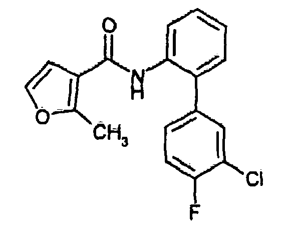 | 100 | 100 |
| 15 | 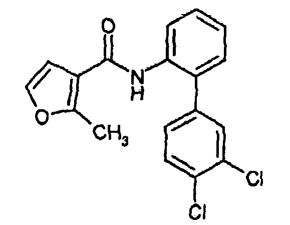 | 100 | 100 |

### Beispiel C

### Alternaria-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer wässrigen Sporensuspension von Alternaria solani inokuliert. Die Pflanzen werden dann in einer Inkubationskabine bei ca. 20°C und 100 % relativer Luftfeuchtigkeit aufgestellt.

2 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C**

| **Alternaria-Test (Tomate) / protektiv** | | | |
|---|---|---|---|
| Wirkstoff | | Aufwandmenge an Wirkstoff in g/ha | Wirkungsgrad in % |
| 2 | 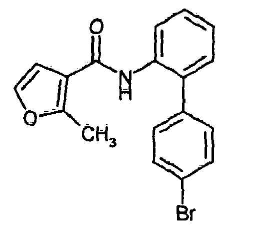 | 100 | 100 |
| 3 | 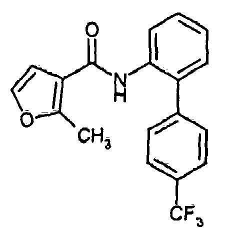 | 100 | 100 |
| 4 | 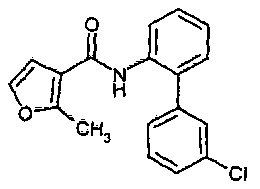 | 100 | 98 |
| 5 | 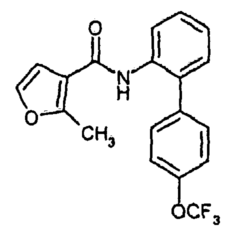 | 100 | 92 |
| 6 | 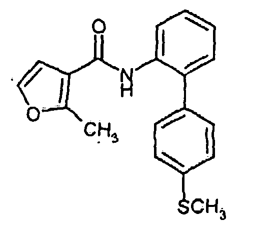 | 100 | 95 |
| 8 | 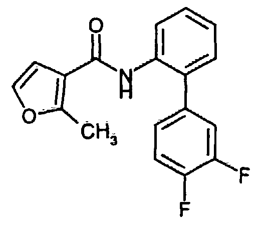 | 100 | 82 |
| 9 | 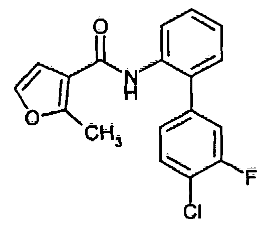 | 100 | 100 |
| 10 | 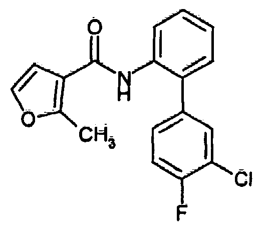 | 100 | 95 |
| 15 | 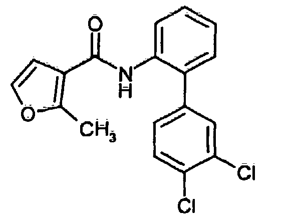 | 100 | 96 |

### Beispiel D

### Sphaerotheca-Test (Gurke) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle D**

| **Sphaerotheca-Test (Gurke) / protektiv** | | | |
|---|---|---|---|
| Wirkstoff | | Aufwandmenge an Wirkstoff in g/ha | Wirkungsgrad in % |
| 2 | 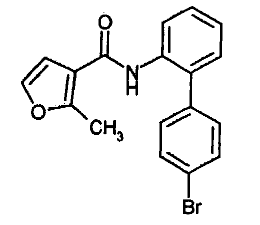 | 750 | 90 |
| 3 | 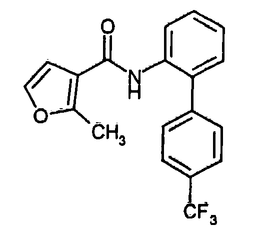 | 750 | 100 |
| 4 | 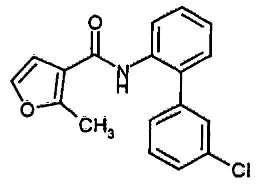 | 750 | 95 |
| 16 | 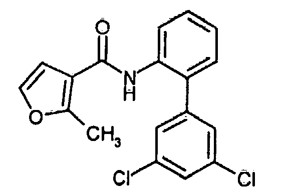 | 750 | 90 |

### Beispiel E

### Pyrenophora teres-Test (Gerste) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Pyrenophora teres besprüht. Die Pflanzen verbleiben 48 h bei 20°C und 100 % relativer Luftfeuchtigkeit in einer Inkubationskabine. Die Pflanzen werden dann in einem Gewächshaus bei einer Temperatur von ca. 20°C und einer relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle E**

| **Pyrenophora teres-Test (Gerste) / protektiv** | | | |
|---|---|---|---|
| Wirkstoff | | Aufwandmenge an Wirkstoff in g/ha | Wirkungsgrad in % |
| 15 | 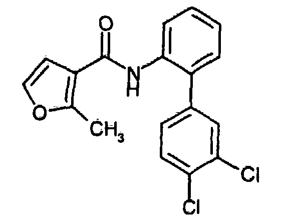 | 500 | 90 |

## Patentansprüche

1. Furancarboxamide der Formel (I) 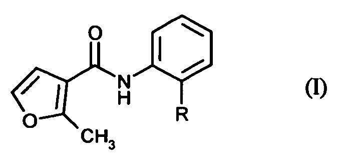 in welcher
R für eine der folgenden Gruppierungen steht, 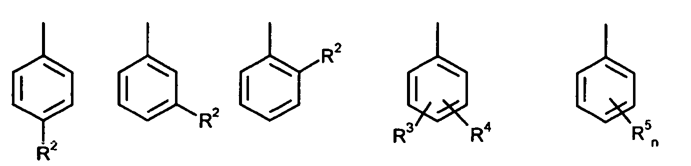
R² für Halogen, Cyano, Nitro, C₁-C₈-Alkyl, C₁-C₈-Alkoxy, C₁-C₈-Alkylthio, C₁-C₆-Halo-genalkyl, C₁-C₆-Halogenalkoxy oder C₁-C₆-Halogenalkylthio mit jeweils 1 bis 13 Fluor-, Chlor- und/oder Bromatomen steht,
R³ und R⁴ unabhängig voneinander für Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogenalkylthio mit jeweils 1 bis 13 Fluor-, Chlor- und/oder Bromatomen stehen,
n für 3, 4 oder 5 steht und
R⁵ für gleiche oder verschiedene Reste aus der Reihe Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogenalkylthio mit jeweils 1 bis 13 Fluor-, Chlor- und/oder Bromatomen steht.

2. Furancarboxamide der Formel (I) gemäß Anspruch 1, in welcher
R für eine der folgenden Gruppierungen steht, 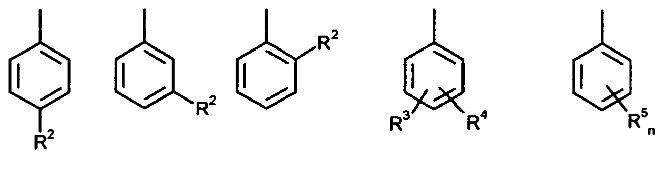
R² für Fluor, Chlor, Brom, Iod, C₁-C₆-Alkyl, C₁-C₆-Alkoxy, C₁-C₆-Alkylthio, C₁-C₄-Halo-genalkyl, C₁-C₄-Halogenalkoxy, C₁-C₄-Halogenalkylthio mit jeweils 1 bis 9 Fluor-, Chlor und/oder Bromatomen steht,
R² außerdem für Cyano steht,
R³ und R⁴ unabhängig voneinander für Fluor, Chlor, Brom, Iod, C₁-C₆-Alkyl, C₁-C₆-Alkoxy, C₁-C₆-Alkylthio, C₁-C₄-Halogenalkyl, C₁-C₄-Halogenalkoxy, C₁-C₄-Halogenalkylthio mit jeweils 1 bis 9 Fluor-, Chlor und/oder Bromatomen stehen,
n für 3, 4 oder 5 steht und
R⁵ für gleiche oder verschiedene Reste aus der Reihe Fluor, Chlor, Brom, Iod, C₁-C₆-Alkyl, C₁-C₆-Alkoxy, C₁-C₆-Alkylthio, C₁-C₄-Halogenalkyl, C₁-C₄-Halogenalkoxy, C₁-C₄-Halogenalkylthio mit jeweils 1 bis 9 Fluor-, Chlor und/oder Bromatomen steht.

3. Furancarboxamide der Formel (I) gemäß Anspruch 1, in welcher
R für eine der folgenden Gruppierungen steht, 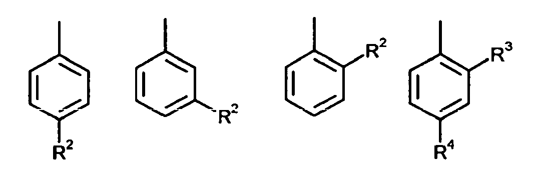 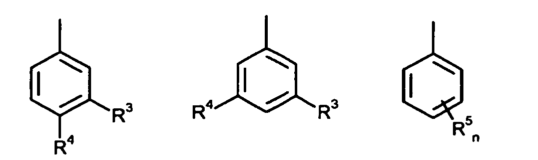
R² für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Di-fluormethyl, Difluorchlormethyl, Difluormethoxy, Trifluormethoxy, Trifluormethylthio, Difluorchlormethylthio steht,
R² außerdem für Cyano steht,
R³ und R⁴ unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen,
n für 3 oder 4 steht und
R⁵ für gleiche oder verschiedene Reste aus der Reihe Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio steht.

4. Furancarboxamide der Formel (I) gemäß Anspruch 1, in welcher
R für eine der folgenden Gruppierungen steht, 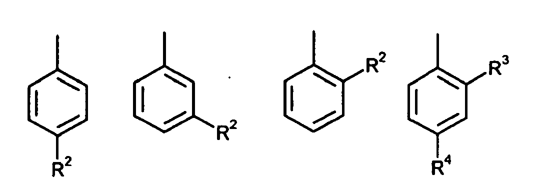 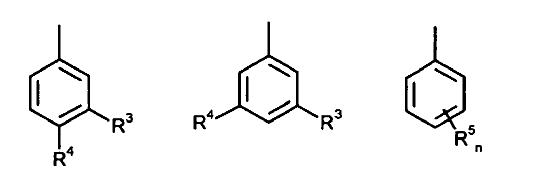
R² für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio steht,
R² außerdem für Cyano steht,
R³ und R⁴ unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio stehen,
n für 3 steht und
R⁵ für gleiche oder verschiedene Reste aus der Reihe Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Trifluormethoxy, Trifluormethylthio steht.

5. Furancarboxamide der Formel (I) gemäß Anspruch 1, in welcher
R für eine der folgenden Gruppierungen steht, 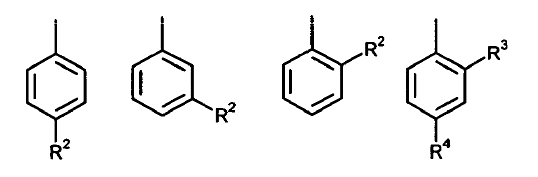 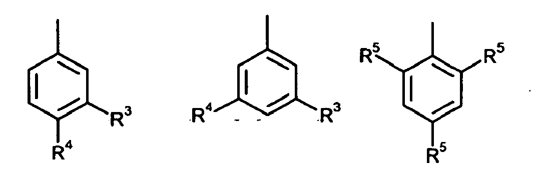
R² für Fluor, Chlor, Brom, t-Butyl, Methoxy, Methylthio, Trichlormethyl, Trifluormethyl, Trifluormethoxy, Trifluormethylthio steht,
R² außerdem für Cyano steht,
R³ und R⁴ unabhängig voneinander für Fluor, Chlor, Methyl, Methoxy, Methylthio, Trifluormethyl, Trifluormethoxy, Trifluormethylthio stehen,
R⁵ für gleiche oder verschiedene Reste aus der Reihe Fluor, Chlor, Methyl, Methoxy, Methylthio, Trifluormethyl, Trifluormethoxy, Trifluormethylthio steht.

6. Furancarboxamide gemäß Anspruch 1 der Formel (I-a) 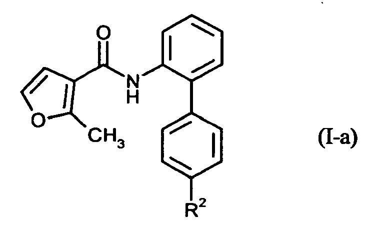 in welcher
R² für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Di-fluormethyl, Difluorchlormethyl, Difluormethoxy, Trifluormethoxy, Trifluormethylthio, Difluorchlormethylthio steht,
R² außerdem für Cyano steht.

7. Furancarboxamide gemäß Anspruch 1 der Formel (I-b) 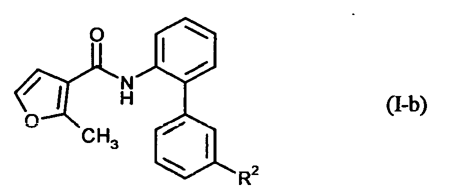 in welcher
R² für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Di-fluormethyl, Difluorchlormethyl, Difluormethoxy, Trifluormethoxy, Trifluormethylthio, Difluorchlormethylthio steht.

8. Furancarboxamide gemäß Anspruch 1 der Formel (I-c) 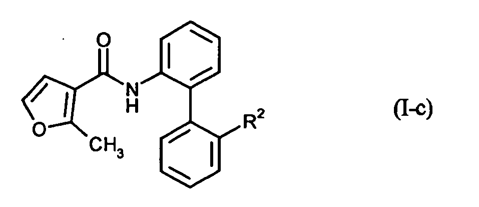 in welcher
R² für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio steht.

9. Furancarboxamide gemäß Anspruch 1 der Formel (I-d) 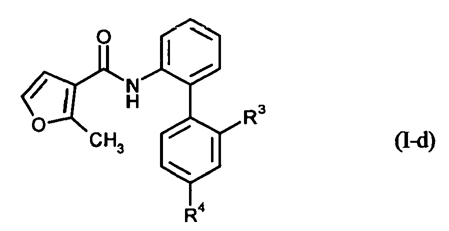 in welcher
R³ und R⁴ unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen.

10. Furancarboxamide gemäß Anspruch 1 der Formel (I-e) 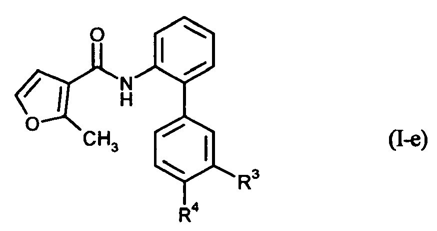 in welcher
R³ und R⁴ unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlormethyl, Trifluormethyl, Difluormethyl, Difluorchlormethyl, Difluormethoxy, Trifluormethoxy, Trifluormethylthio, Difluorchlormethylthio stehen.

11. Furancarboxamide gemäß Anspruch 1 der Formel (I-f) 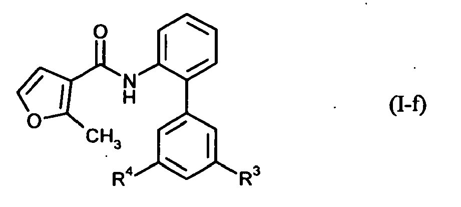 in welcher
R³ und R⁴ unabhängig voneinander für Fluor, Chlor, Brom, Methyl, Ethyl, n-Propyl, i-Propyl, n-Butyl, i-Butyl, s-Butyl, t-Butyl, Methoxy, Ethoxy, Methylthio, Ethylthio, Trichlonnethyl, Trifluormethyl, Difluormethyl, Difluorchlormethyl, Difluonnethoxy, Trifluormethoxy, Trifluormethylthio, Difluorchlormethylthio stehen.

12. Verfahren zum Herstellen von Furancarboxamiden Formel (I) gemäß Anspruch 1, **dadurch gekennzeichnet, dass** man
a) Carbonsäure-Derivate der Formel (II) 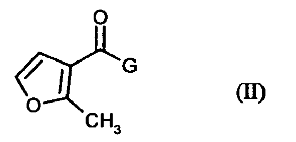 in welcher G für Halogen, Hydroxy oder C₁-C₆-Alkoxy steht,
mit Anilin-Derivaten der Formel (III) 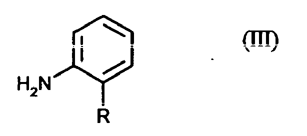 in welcher R die in Anspruch 1 angegebenen Bedeutungen hat,
gegebenenfalls in Gegenwart eines Katalysators, gegebenenfalls in Gegenwart eines Säurebindemittels und gegebenenfalls in Gegenwart eines Verdünnungsmittels umsetzt,
oder
b) Carboxamid-Derivate der Formel (IV) 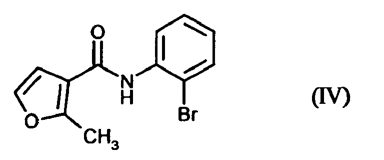 mit Boronsäure-Derivaten der Formel (V) 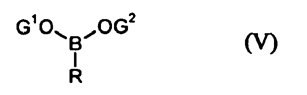 in welcher
R die in Anspruch 1 angegebenen Bedeutungen hat und
G¹ und G² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt,
oder
c) Carboxamid-Boronsäure-Derivate der Formel (VI) 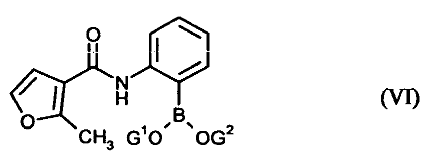 in welcher
G¹ und G² jeweils für Wasserstoff oder zusammen für Tetramemylethylen stehen,
mit Phenyl-Derivaten der Formel (VII) 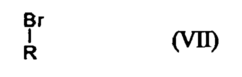 in welcher R die in Anspruch 1 angegebenen Bedeutungen hat,
in Gegenwart eines Katalysators, gegebenenfalls in Gegenwart eines Säurebindemittels und gegebenenfalls in Gegenwart eines Verdünnungsmittels umsetzt,
oder
d) Carboxamid-Derivate der Formel (IV) 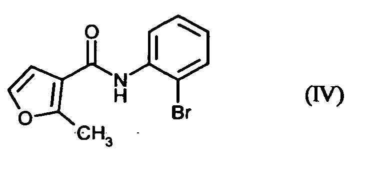 mit Phenyl-Derivaten der Formel (VII) 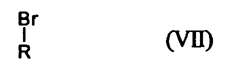 in welcher R die in Anspruch 1 angegebenen Bedeutungen hat,
in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

13. Mittel zur Bekämpfung unerwünschter Mikroorganismen, **gekennzeichnet durch** einen Gehalt an mindestens einem Furancarboxamid der Formel (I) gemäß Anspruch 1 neben Streckmitteln und/oder oberflächenaktiven Stoffen.

14. Verwendung von Furancarboxamiden der Formel (I) gemäß Anspruch 1 zur Bekämpfung unerwünschter Mikroorganismen.

15. Verfahren zur Bekämpfung unerwünschter Mikroorganismen, **dadurch gekennzeichnet, dass** man Furancarboxamide der Formel (I) gemäß Anspruch 1 auf die Mikroorganismen und/oder deren Lebensraum ausbringt.

16. Verfahren zur Herstellung von Mitteln zur Bekämpfung unerwünschter Mikroorganismen, **dadurch gekennzeichnet, dass** man Furancarboxamide der Formel (I) gemäß Anspruch 1 mit Streckmitteln und/oder oberflächenaktiven Stoffen vermischt.

## Claims

1. Furancarboxamides of the formula (I) 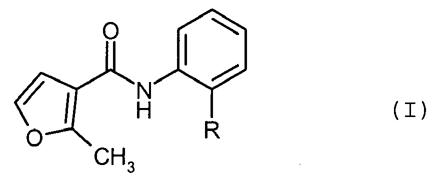 in which
R represents one of the groupings below, 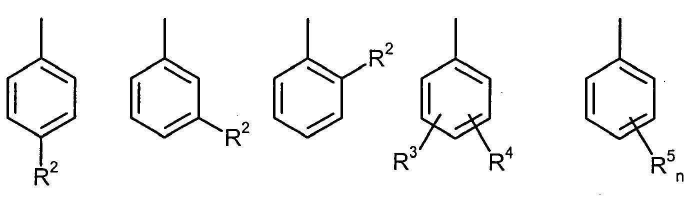
R² represents halogen, cyano, nitro, C₁-C₈-alkyl, C₁-C₈-alkoxy, C₁-C₈-alkylthio, C₁-C₆-haloalkyl, C₁-C₆-haloalkoxy or C₁-C₆-haloalkylthio having in each case 1 to 13 fluorine, chlorine and/or bromine atoms,
R³ and R⁴ independently of one another represent halogen, cyano, nitro, C₁-C₈-alkyl, C₁-C₈-alkoxy, C₁-C₈-alkylthio, C₁-C₆-haloalkyl, C₁-C₆-haloalkoxy or C₁-C₆-haloalkylthio having in each case 1 to 13 fluorine, chlorine and/or bromine atoms,
n represents 3, 4 or 5 and
R⁵ represents identical or different radicals from the group consisting of halogen, cyano, nitro, C₁-C₈-alkyl, C₁-C₈-alkoxy, C₁-C₈-alkyl-thio, C₁-C₆-haloalkyl, C₁-C₆-haloalkoxy or C₁-C₆-haloalkylthio having in each case 1 to 13 fluorine, chlorine and/or bromine atoms.

2. Furancarboxamides of the formula (I) according to Claim 1 in which
R represents one of the groupings below, 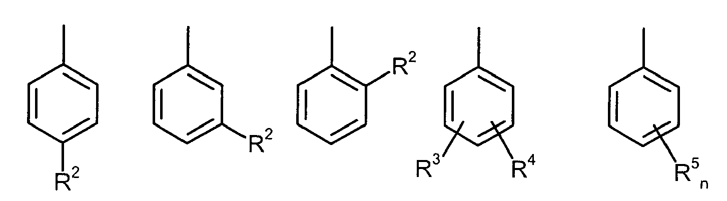
R² represents fluorine, chlorine, bromine, iodine, C₁-C₆-alkyl, C₁-C₆-alkoxy, C₁-C₆-alkylthio, C₁-C₄-haloalkyl, C₁-C₄-haloalkoxy, C₁-C₄-haloalkylthio having in each case 1 to 9 fluorine, chlorine and/or bromine atoms,
R² furthermore represents cyano,
R³ and R⁴ independently of one another represent fluorine, chlorine, bromine, iodine, C₁-C₆-alkyl, C₁-C₆-alkoxy, C₁-C₆-alkylthio, C₁-C₄-haloalkyl, C₁-C₄-haloalkoxy, C₁-C₄-haloalkylthio having in each case 1 to 9 fluorine, chlorine and/or bromine atoms,
n represents 3, 4 or 5 and
R⁵ represents identical or different radicals from the group consisting of fluorine, chlorine, bromine, iodine, C₁-C₆-alkyl, C₁-C₆-alkoxy, C₁-C₆-alkylthio, C₁-C₄-haloalkyl, C₁-C₄-haloalkoxy, C₁-C₄-haloalkylthio having in each case 1 to 9 fluorine, chlorine and/or bromine atoms.

3. Furancarboxamides of the formula (I) according to Claim 1 in which
R represents one of the groupings below 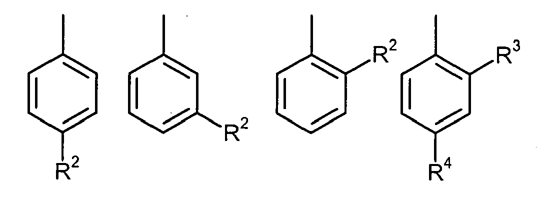 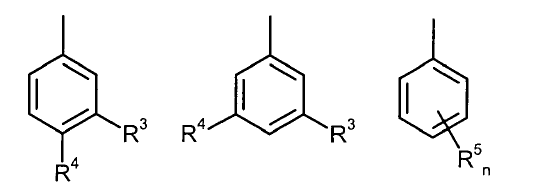
R² represents fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio,
R² furthermore represents cyano,
R³ and R⁴ independently of one another represent fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio,
n represents 3 or 4 and
R⁵ represents identical or different radicals from the group consisting of fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

4. Furancarboxamides of the formula (I) according to Claim 1 in which
R represents one of the groupings below 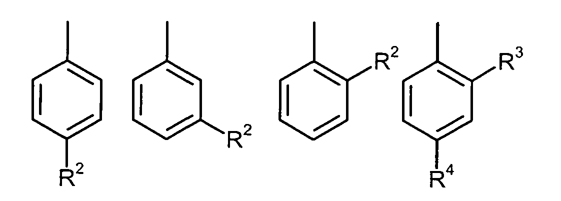 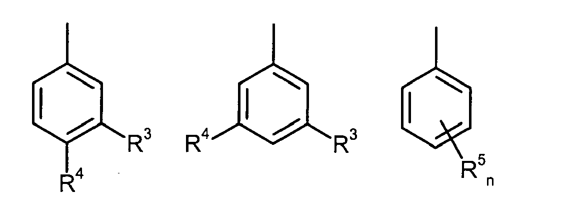
R² represents fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, trifluoromethoxy, trifluoromethylthio,
R² furthermore represents cyano,
R³ and R⁴ independently of one another represent fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, trifluoromethoxy, trifluoromethylthio,
n represents 3 and
R⁵ represents identical or different radicals from the group consisting of fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, trifluoromethoxy, trifluoromethylthio.

5. Furancarboxamides of the formula (I) according to Claim 1 in which
R represents one of the groupings below 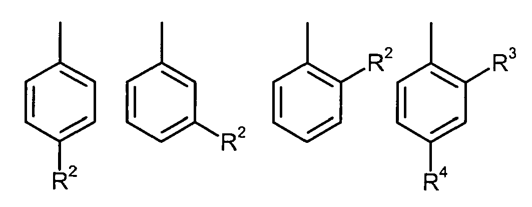 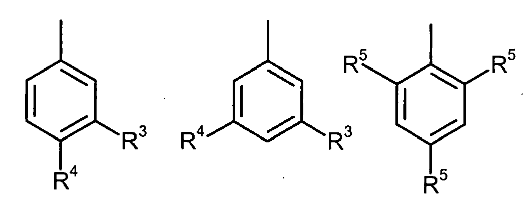
R² represents fluorine, chlorine, bromine, t-butyl, methoxy, methylthio, trichloromethyl, trifluoromethyl, trifluoromethoxy, trifluoromethylthio,
R² furthermore represents cyano,
R³ and R⁴ independently of one another represent fluorine, chlorine, methyl, methoxy, methylthio, trifluoromethyl, trifluoromethoxy, trifluoromethylthio,
R⁵ represents identical or different radicals from the group consisting of fluorine, chlorine, methyl, methoxy, methylthio, trifluoromethyl, trifluoromethoxy, trifluoromethylthio.

6. Furancarboxamides according to Claim 1 of the formula (I-a) 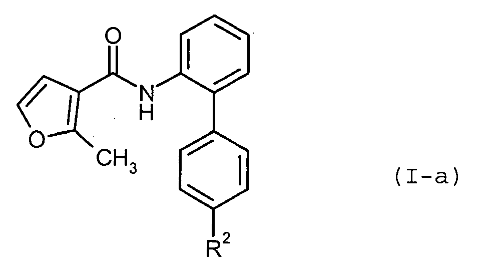 in which
R² represents fluorine, chlorine, bromine methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio,
R² furthermore represents cyano.

7. Furancarboxamides according to Claim 1 of the formula (I-b) 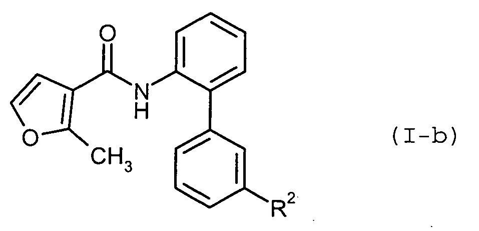 in which
R² represents fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

8. Furancarboxamides according to Claim 1 of the formula (I-c) 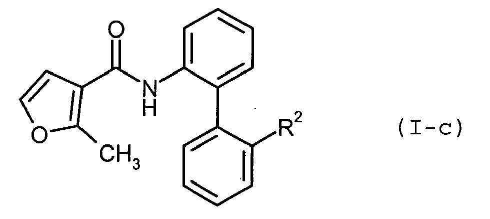 in which
R² represents fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

9. Furancarboxamides according to Claim 1 of the formula (I-d) 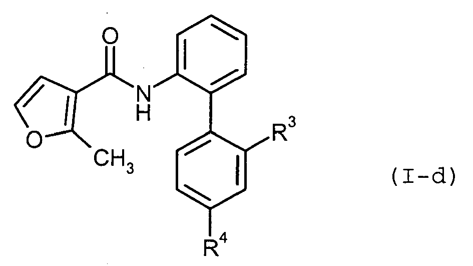 in which
R³ and R⁴ independently of one another represent fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

10. Furancarboxamides according to Claim 1 of the formula (I-e) 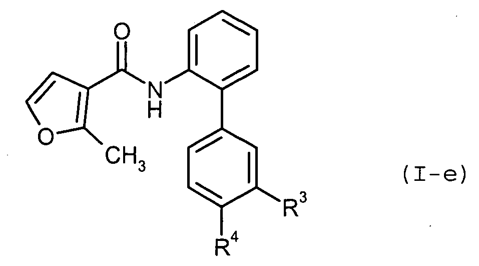 in which
R³ and R⁴ independently of one another represent fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

11. Furancarboxamides according to Claim 1 of the formula (I-f) 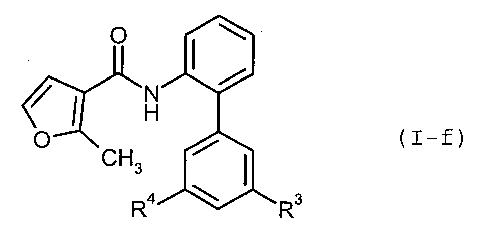 in which
R³ and R⁴ independently of one another represent fluorine, chlorine, bromine, methyl, ethyl, n-propyl, i-propyl, n-butyl, i-butyl, s-butyl, t-butyl, methoxy, ethoxy, methylthio, ethylthio, trichloromethyl, trifluoromethyl, difluoromethyl, difluorochloromethyl, difluoromethoxy, trifluoromethoxy, trifluoromethylthio, difluorochloromethylthio.

12. Process for preparing furancarboxamides of the formula (I) according to Claim 1, **characterized in that**
a) carboxylic acid derivatives of the formula (II) 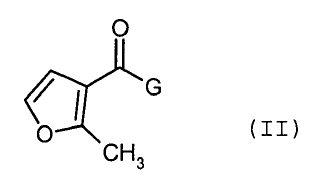 in which G represents halogen, hydroxyl or C₁-C₆-alkoxy,
are reacted with aniline derivatives of the formula (III) 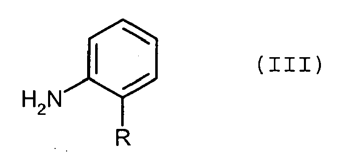 in which R is as defined in Claim 1,
if appropriate in the presence of a catalyst, if appropriate in the presence of an acid binder and if appropriate in the presence of a diluent,
or
b) carboxamide derivatives of the formula (IV) 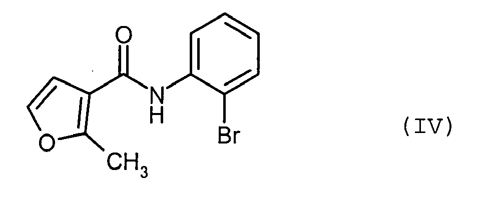 are reacted with boronic acid derivatives of the formula (V) 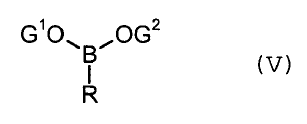 in which
R is as defined in Claim 1 and
G¹ and G² each represent hydrogen or together represent tetramethylethylene,
in the presence of a catalyst, if appropriate in the presence of an acid binder and if appropriate in the presence of a diluent,
or
c) carboxamide boronic acid derivatives of the formula (VI) 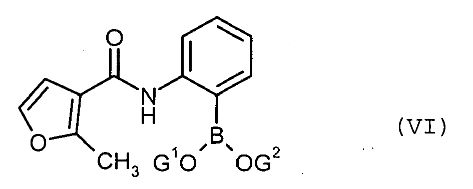 in which
G¹ and G² each represent hydrogen or together represent tetramethylethylene
are reacted with phenyl derivatives of the formula (VII) 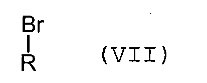 in which R is as defined in Claim 1,
in the presence of a catalyst, if appropriate in the presence of an acid binder and if appropriate in the presence of a diluent,
or
d) carboxamide derivatives of the formula (IV) 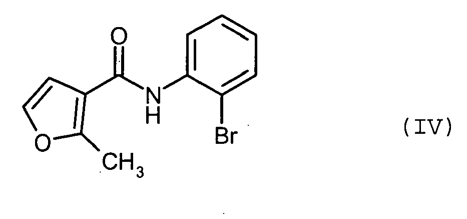 are reacted with phenyl derivatives of the formula (VII) 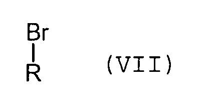
in which R is as defined in Claim 1,
in the presence of a palladium or platinum catalyst and in the presence of 4,4,4',4',5,5,5',5'-octamethyl-2,2'-bis-1,3,2-dioxaborolane, if appropriate in the presence of an acid binder and if appropriate in the presence of a diluent.

13. Compositions for controlling unwanted micro-organisms, **characterized in that** they comprise at least one furancarboxamide of the formula (I) according to Claim 1, in addition to extenders and/or surfactants.

14. Use of furancarboxamides of the formula (I) according to Claim 1 for controlling unwanted micro-organisms.

15. Method for controlling unwanted micro-organisms, **characterized in that** furancarboxamides of the formula (I) according to Claim 1 are applied to the micro-organisms and/or their habitat.

16. Process for preparing compositions for controlling unwanted micro-organisms, **characterized in that** furancarboxamides of the formula (I) according to Claim 1 are mixed with extenders and/or surfactants.

## Revendications

1. Furanne-carboxamides de formule (I) 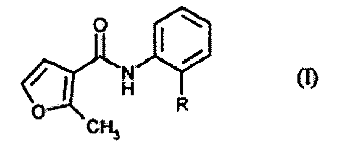 dans laquelle
R représente l'un des groupements suivants 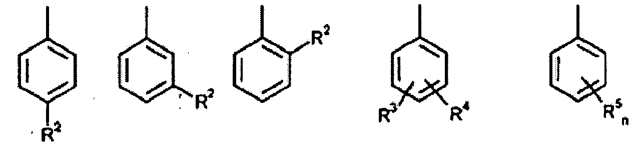
R² représente un halogène, un groupe cyano, nitro, un reste alkyle en C₁ à C₈, alkoxy en C₁ à C₈, alkylthio en C₁ à C₈, halogénalkyle en C₁ à C₆, halogénalkoxy en C₁ à C₆ ou halogénalkylthio en C₁ à C₆ ayant dans chaque cas 1 à 13 atomes de fluor, de chlore et/ou de brome,
R³ et R⁴ représentent, indépendamment l'un de l'autre, un halogène, un groupe cyano, nitro, un reste alkyle en C₁ à C₈, alkoxy en C₁ à C₈, alkylthio en C₁ à C₈, halogénalkyle en C₁ à C₆, halogénalkoxy en C₁ à C₆ ou halogénalkylthio en C₁ à C₆ ayant dans chaque cas 1 à 13 atomes de fluor, de chlore et/ou de brome,
n a la valeur 3, 4 ou 5 et
R⁵ représente des restes identiques ou différents de la série halogéno, cyano, nitro, alkyle en C₁ à C₈, alkoxy en C₁ à C₈, alkylthio en C₁ à C₈, halogénalkyle en C₁ à C₆, halogénalkoxy en C₁ à C₆ ou halogénalkylthio en C₁ à C₆ ayant dans chaque cas 1 à 13 atomes de fluor, de chlore et/ou de brome.

2. Furanne-carboxamides de formule (I) suivant la revendication 1, dans laquelle
R représente l'un des groupements suivants : 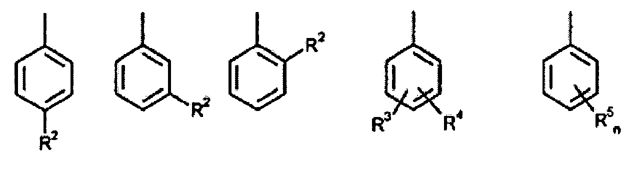
R² représente le fluor, le chlore, le brome, l'iode, un reste alkyle en C₁ à C₆, alkoxy en C₁ à C₆, alkylthio en C₁ à C₆, halogénalkyle en C₁ à C₄, halogénalkoxy en C₁ à C₄, halogénalkylthio en C₁ à C₄, avec dans chaque cas 1 à 9 atomes de fluor, de chlore et/ou de brome,
R² représente en outre un groupe cyano,
R³ et R⁴ représentent, indépendamment l'un de l'autre, le fluor, le chlore, le brome, l'iode, un reste alkyle en C₁ à C₆, alkoxy en C₁ à C₆, alkylthio en C₁ à C₆, halogénalkyle en C₁ à C₄, halogénalkoxy en C₁ à C₄, halogénalkylthio en C₁ à C₄, avec dans chaque cas 1 à 9 atomes de fluor, de chlore et/ou de brome,
n a la valeur 3, 4 ou 5 et
R⁵ représente des restes, identiques ou différents, de la série fluoro, chloro, bromo, iodo, alkyle en C₁ à C₆, alkoxy en C₁ à C₆, alkylthio en C₁ à C₆, halogénalkyle en C₁ à C₄, halogénalkoxy en C₁ à C₄, halogénalkylthio en C₁ à C₄, avec dans chaque cas 1 à 9 atomes de fluor, de chlore et/ou de brome.

3. Furanne-carboxamides de formule (I) suivant la revendication 1, dans laquelle
R représente l'un des groupements suivants : 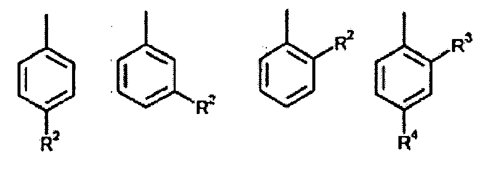 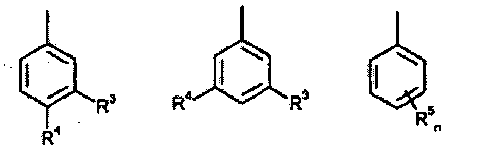
R² représente le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio,
R² représente en outre un groupe cyano,
R³ et R⁴ représentent, indépendamment l'un de l'autre, le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio,
n a la valeur 3 ou 4 et
R⁵ représente des restes, identiques ou différents, de la série fluor, chlore, brome, méthyle, éthyle, n-propyle, isopropyle, n-butyle, isobutyle, sec. -butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

4. Furanne-carboxamides de formule (I) suivant la revendication 1, dans laquelle
R représente l'un des groupements suivants : 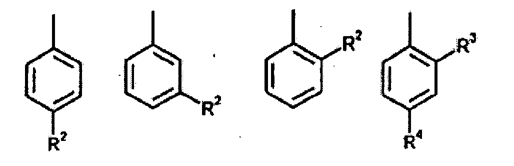 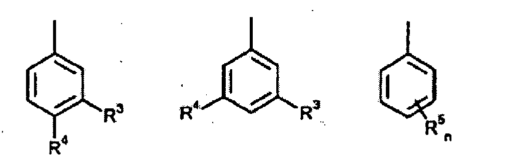
R² représente le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, trifluorométhoxy, trifluorométhylthio,
R² représente en outre un groupe cyano,
R³ et R⁴ représentent, indépendamment l'un de l'autre, le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, trifluorométhoxy, trifluorométhylthio,
n a la valeur 3 et
R⁵ représente des restes, identiques ou différents, de la série fluoro, chloro, bromo, méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, trifluorométhoxy, trifluorométhylthio.

5. Furanne-carboxamides de formule (I) suivant la revendication 1, dans laquelle
R représente l'un des groupements suivants : 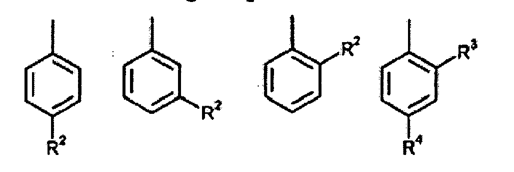 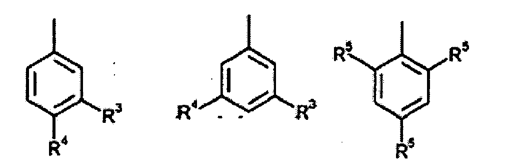
R² représente le fluor, le chlore, le brome, un reste tertiobutyle, méthoxy, méthylthio, trichlorométhyle, trifluorométhyle, trifluorométhoxy, trifluorométhylthio,
R² représente en outre un groupe cyano,
R³ et R⁴ représentent, indépendamment l'un de l'autre, le fluor, le chlore, un reste méthyle, méthoxy, méthylthio, trifluorométhyle, trifluorométhoxy, trifluorométhylthio,
R⁵ représente des restes, identiques ou différents, de la série fluoro, chloro, méthyle, méthoxy, méthylthio, trifluorométhyle, trifluorométhoxy, trifluorométhylthio.

6. Furanne-carboxamides suivant la revendication 1, de formule (I-a) 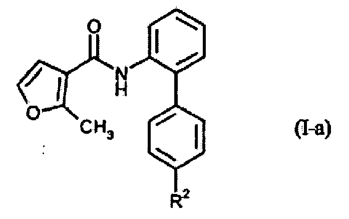 dans laquelle
R² représente le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio,
R² représente en outre un groupe cyano.

7. Furanne-carboxamides suivant la revendication 1, de formule (I-b) 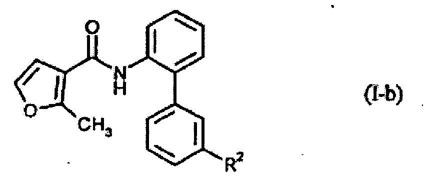 dans laquelle
R² représente le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

8. Furanne-carboxamides suivant la revendication 1, de formule (I-c) 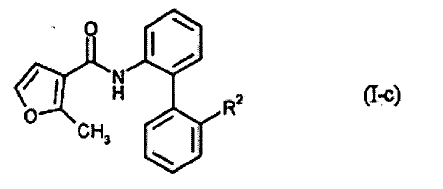 dans laquelle
R² représente le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

9. Furanne-carboxamides suivant la revendication 1, de formule (I-d) 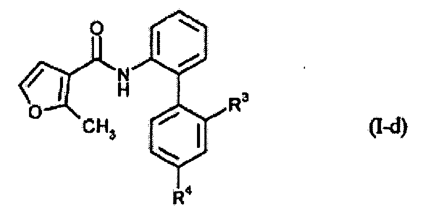 dans laquelle
R³ et R⁴ représentent, indépendamment l'un de l'autre, le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

10. Furanne-carboxamides suivant la revendication 1, de formule (I-e) 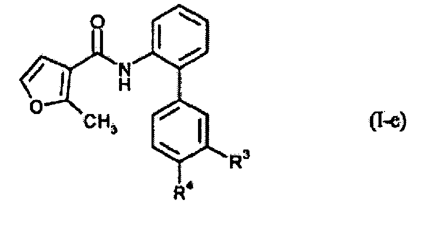 dans laquelle
R³ et R⁴ représentent, indépendamment l'un de l'autre, le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

11. Furanne-carboxamides suivant la revendication 1, de formule (I-f) 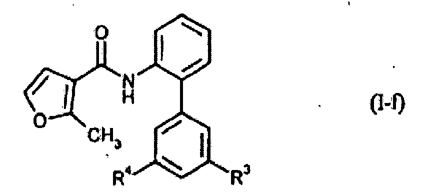 dans laquelle
R³ et R⁴ représentent, indépendamment l'un de l'autre, le fluor, le chlore, le brome, un reste méthyle, éthyle, n-propyle, isopropyle, n-butyle, isobutyle, sec.-butyle, tertiobutyle, méthoxy, éthoxy, méthylthio, éthylthio, trichlorométhyle, trifluorométhyle, difluorométhyle, difluorochlorométhyle, difluorométhoxy, trifluorométhoxy, trifluorométhylthio, difluorochlorométhylthio.

12. Procédé de production de furanne-carboxamides de formule (I) suivant la revendication 1, **caractérisé en ce que**
a) on fait réagir des dérivés d'acides carboxyliques de formule (II) 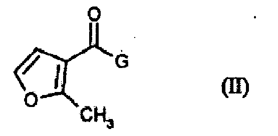 dans laquelle G représente un halogène, un groupe hydroxy ou un reste alkoxy en C₁ à C₆,
avec des dérivés d'aniline de formule (III) 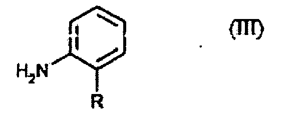 dans laquelle R a les définitions indiquées dans la revendication 1,
éventuellement en présence d'un catalyseur, le cas échéant en présence d'un accepteur d'acide et, éventuellement, en présence d'un diluant,
ou bien
b) on fait réagir des dérivés de carboxamides de formule (IV) 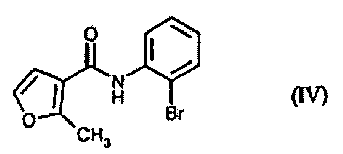 avec des dérivés d'acides boroniques de formule (V) 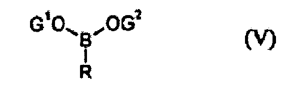 dans laquelle R a les définitions indiquées dans la revendication 1, et
G¹ et G² représentent chacun l'hydrogène ou forment ensemble un groupe tétraméthyléthylène,
en présence d'un catalyseur, éventuellement en présence d'un accepteur d'acide et, le cas échéant, en présence d'un diluant,
ou bien
c) on fait réagir des dérivés d'acides carboxamide-boroniques de formule (VI) 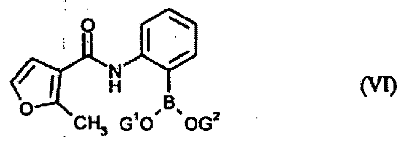 dans laquelle
G¹ et G² représentent chacun l'hydrogène ou forment ensemble un groupe tétraméthyléthylène,
avec des dérivés phényliques de formule (VII) 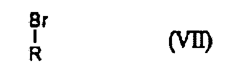 dans laquelle R a les définitions indiquées dans la revendication 1,
en présence d'un catalyseur, éventuellement en présence d'un accepteur d'acide et, le cas échéant, en présence d'un diluant,
ou bien
d) on fait réagir des dérivés de carboxamides de formule (IV) 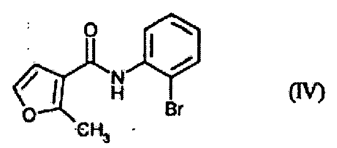 avec des dérivés phényliques de formule (VII) 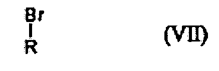 dans laquelle R a les définitions indiquées dans la revendication 1,
en présence d'un catalyseur au palladium ou au platine et en présence de 4,4,4',4',5,5,5',5'-octaméthyl-2,2'-bis-1,3,2-dioxaborolanne, éventuellement en présence d'un accepteur d'acide et, le cas échéant, en présence d'un diluant.

13. Composition destinée à la lutte contre des micro-organismes indésirables, **caractérisée par** une teneur en au moins un furanne-carboxamide de formule (I) suivant la revendication 1, à côté de diluants et/ou de substances tensioactives.

14. Utilisation de furanne-carboxamides de formule (I) suivant la revendication 1 pour la lutte contre des micro-organismes indésirables.

15. Procédé pour combattre des micro-organismes indésirables, **caractérisé en ce qu'**on épand des furanne-carboxamides de formule (I) suivant la revendication 1 sur les micro-organismes et/ou sur leur milieu.

16. Procédé de préparation de compositions destinées à la lutte contre des micro-organismes indésirables, **caractérisé en ce qu'**on mélange des furanne-carboxamides de formule (I) suivant la revendication 1 avec des diluants et/ou des agents tensioactifs.
